# FASCICULE DE BREVET EUROPEEN

(11) **EP 0 541 407 B1**
(45) Date de publication et mention de la délivrance du brevet: **26.02.1997**
(21) Numéro de dépôt: 92402631.3
(22) Date de dépôt: 25.09.1992
(51) Int. Cl.: C07D 453/06, C07D 209/52, C07D 209/42, C07D 217/26, C07D 403/06, C07D 417/06, A61K 31/435, A61K 31/40, C07D 277/06, C07D 519/00, C07D 487/08

(54) **Dérivés bicycliques azotés, leur procédé de préparation et les compositions pharmaceutiques qui les contiennent**
Stickstoff enthaltende bizyklische Derivate, Verfahren zu ihrer Herstellung und diese enthaltende pharmazeutische Zubereitungen
Nitrogen containing bicyclic derivatives, method for their preparation and pharmaceutical compositions containing them

(30) Priorité: 27.09.1991 FR 9111893
(43) Date de publication de la demande: 12.05.1993
(73) Titulaire: ADIR ET COMPAGNIE, 92415 Courbevoie Cédex (FR)
(72) Inventeur: Vincent, Michel, F-92220 Bagneux (FR); Remond, Georges, F-78000 Versailles (FR); Portevin, Bernard, F-78990 Elancourt (FR); Herve, Yolande, F-92800 Puteaux (FR); Lepagnol, Jean, F-28210 Chaudon (FR); de Nanteuil, Guillaume, F-92150 Suresnes (FR)

(56) Documents cités:
- EP-A- 0 320 753
- JOURNAL OF ENZYME INHIBITION vol. 5, no. 1, 1991, CHUR, CH pages 51 - 75 NAOKI HIGUCHI ET AL. 'Synthesis and inhibitory activity of acyl-peptidyl-pyrrolidine derivatives toward post-proline cleaving enzyme; a study of subsite specificity'
- CHEMICAL ABSTRACTS, vol. 112, 1990, Columbus, Ohio, US; abstract no. 682e, 'Prolyl endopeptidase-inhibiting thiazolidines for treatment of amnesia and dementia' page 66 ; J. Biochem., vol. 104, 1986, pages 580-586

## Description

La présente invention concerne de nouveaux dérivés bicycliques azotés, leur procédé de préparation et les compositions pharmaceutiques qui les contiennent.

Le vieillissement de la population par allongement de la durée de vie rend de plus en plus important le problème du vieillissement cérébral et des démences séniles liées à l'âge. C'est pourquoi, la recherche de nouveaux composés thérapeutiques pouvant s'opposer aux déficits de la mémoire et aux troubles neuropsychocomportementaux de la sénescence est devenue prioritaire.

A côté des neurotransmetteurs classiques (acétylcholine, norepinephrine) impliqués dans les fonctions de mémoire, il y a des neuropeptides tels que la vasopressine (AVP) ou la thyrotropin releasing hormone (TRH) qui exercent également des effets facilitateurs mnésiques en jouant un rôle de neuromodulation en plus de leur rôle périphérique ou endocrinien (Science, 211, 601, 1981).

Récemment, des études ont montré que les taux cérébraux de ces peptides diminuent significativement chez les patients atteints de démence sénile (Neurobiology, 36, 1133, 1986).

Encore plus récemment, il a été observé que l'activité de la prolylendopeptidase, enzyme-clé du catabolisme de ces peptides, augmente considérablement (+ 100 %) dans le cerveau des malades d'Alzheimer (Experientia, 46, 94, 1990). C'est pourquoi, il a été suggéré et démontré que des inhibiteurs de la prolyl-endopeptidase peuvent s'opposer aux déficits de la mémoire en favorisant l'effet promnésique de certains neuropeptides et notamment de la vasopressine. Ces résultats ont été observés notamment lors d'amnésie expérimentale à la scopolamine. Pour les composés inhibiteurs étudiés, une excellente corrélation entre l'effet inhibiteur de la prolyl-endopeptidase et l'effet facilitateur de l'apprentissage a été rapportée (EP 321 956).

Il était donc particulièrement intéressant de synthétiser de nouveaux composés possédant une activité inhibitrice de la prolyl-endopeptidase (ou post-prolyl cleaving enzyme : PPCE).

Les composés de la présente invention, outre le fait qu'ils soient nouveaux, se sont montrés particulièrement intéressants par l'intensité et la durée de leurs propriétés à inhiber la prolyl-endopeptidase donc à éviter la dégradation des neuropeptides naturels impliqués dans les fonctions mnésiques. Leur activité est supérieure tant in vitro qu'in vivo à celle de composés décrits dans l'art antérieur comme par exemple les composés décrits dans les brevets EP 320 753 EP 321 956 et EP 345 428 en tant qu'inhibiteurs de PPCE ou dans J. Biochem. 104, 580-586, 1988. Elle est également nettement supérieure à celle de nootropes de référence connus également pour inhiber l'activité de la PPCE comme par exemple les composés décrits dans le brevet EP 288 685.

Les composés de l'invention sont donc utiles pour la prévention et le traitement d'une part des troubles comportementaux, notamment de la mémoire, associés au vieillissement et aux maladies dégénératives neuronales aigües ou chroniques et d'autre part des troubles psychiques associés à l'anxiété et la dépression.

L'invention concerne plus particulièrement de nouveaux dérivés bicycliques azotés, répondant à la formule générale (I) : 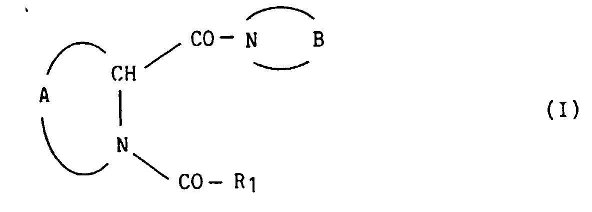 dans laquelle :
- A: représente avec les atomes de carbone et d'azote auquel il est attaché l'hétérocycliques suivant : 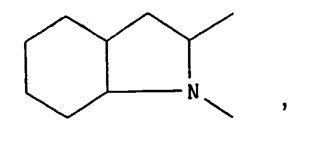
- B: représente avec l'atome d'azote auquel il est attaché l'hétérocycle suivant : 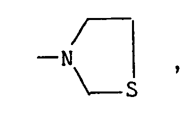
- R₁: représente un groupement cycloalkyle (C₃), étant substitué par un groupement phényle,
leurs énantiomères, diastéréoisomères et épimères ainsi que leurs sels d'addition à un acide pharmaceutiquement acceptable.

Parmi les acides pharmaceutiquement acceptables, on peut citer à titre non limitatif les acides chlorhydrique, sulfurique, tartrique, maléique, fumarique, méthane sulfonique, camphorique, etc...

L'invention s'étend également au procédé de préparation des composés de formule (I) caractérisé en ce que l'on fait réagir un acide de formule (II), dont on a éventuellement séparé les isomères par une technique classique de séparation : 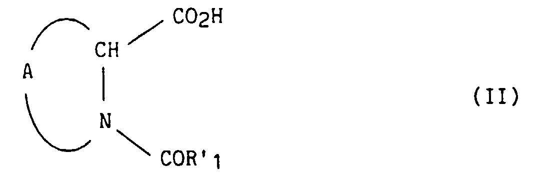 dans laquelle A a la même signification que dans la formule (I) et R′₁ représente un groupement alkoxy (C₁-C₆) linéaire ou ramifié ou benzyloxy,
sur une amine de formule (III), dont on a éventuellement séparé les isomères par une technique classique de séparation, selon une technique de couplage peptidique comme celle décrite par W. KONIG et R. GEIGER (Ber, 103, 788, 1970) : 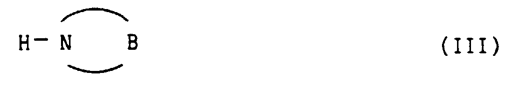 dans laquelle B a la même signification que dans la formule (I),
pour conduire au composé de formule (I/a), cas particulier des composés de formule (I) : 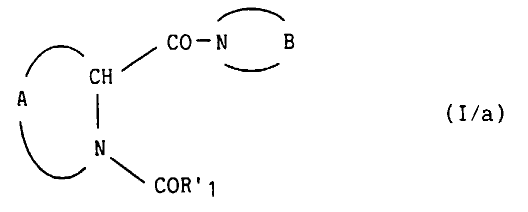 dans laquelle A, B et R′₁ ont la même signification que précédemment,
que l'on déprotège, si on le souhaite, par une technique classique de déprotection,
pour conduire à l'amine de formule (IV) : 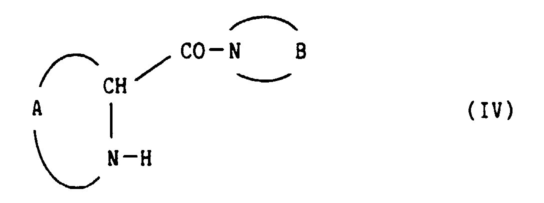 dans laquelle A et B ont la même signification que dans la formule (I),
que l'on fait réagir avec un acide de formule (V) en présence d'un agent de couplage classique de la synthèse peptidique :

HO₂C-R˝₁ (V)

dans laquelle :
- R˝₁: a la même signification que R₁ à l'exception du cas où R₁ représente R′₁
pour conduire au composé de formule (I/b), cas particulier des composés de formule (I) : 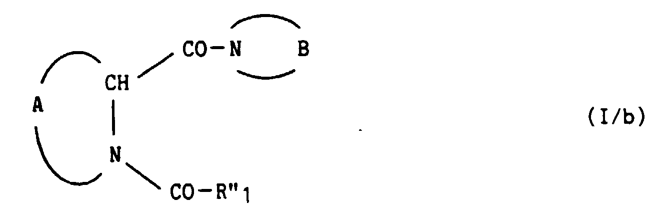 dans laquelle A, B et R˝₁ ont la même signification que précédemment,
dérivés de formule (I/a) et (I/b) que l'on purifie, le cas échéant, par une technique classique de purification, dont on sépare, si on le souhaite, les isomères par une technique classique de séparation et que l'on transforme, si nécessaire, en leurs sels d'addition à un acide pharmaceutiquement acceptable.

Les composés de l'invention possèdent des propriétés pharmacologiques très intéressantes. Ils inhibent fortement l'activité de la prolyl-endopeptidase ce qui les rend utiles pour le traitement des désordres mnésiques et cognitifs et des troubles neurocomportementaux associés au vieillissement et aux maladies dégénératives du système nerveux, aigües ou chroniques comme la maladie d'Alzheimer, de Pick, de Korsakoff, l'accident vasculaire cérébral, le traumatisme spinal ou la sclérose amyotrophique latérale. Ils sont de la même manière utiles dans le traitement des désordres psychiques assoicés à l'anxiété et à la dépression.

La présente invention a également pour objet les compositions pharmaceutiques renfermant comme principe actif au moins un composé de formule générale (I) ou un de ses sels d'addition à un acide pharmacologiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie varie selon l'âge et le poids du patient, la nature et la sévérité de l'affection ainsi que la voie d'administration.

Celle-ci peut être orale, nasale, rectale ou parentérale. D'une manière générale, la posologie unitaire s'échelonne entre 0,5 et 100 mg pour un traitement en 1 à 3 prises par 24 heures.

Les exemples suivants illustrent l'invention et ne la limitent en aucune façon.

Les préparations A à N ne conduisent pas aux composés de l'invention mais à des produits de départ utiles dans la préparation des dérivés de formule (I).

### PREPARATION A : Acide 3,3-dicyclopropylpropanoïque

### STADE A : 3,3-Dicyclopropylpropionitrile

A 40 g de cyanure de sodium dans 45 ml de diméthylsulfoxide (DMSO), sous agitation et à 95°C, sont additionnés lentement 209 g de tosylate de 2,2-dicyclopropyléthanol (préparé à partir de 2,2-dicyclopropyléthanol et de chlorure de paratoluènesulfonyl) en solution dans 45 ml de DMSO. Le chauffage est maintenu une heure. Après filtration du précipité et lavage par de l'éther éthylique, les filtrats sont regroupés, lavés par une solution saturée de chlorure de sodium, séchés et évaporés. Le produit attendu est obtenu après distillation sous vide.
Rendement : 90 %
Point d'ébullition : 95-100°C (15 mm/Hg)
Indice de réfraction : n_{D}^{22,5} = 1,457

### STADE B : Acide 3,3-dicyclopropylpropanoïque

45 g de potasse sont dissous dans 65 ml d'eau. 100 ml de diéthylèneglycol sont ajoutés à la solution précédente puis 30 g du produit obtenu au stade A. L'ensemble est porté à reflux jusqu'à la fin du dégagement d'ammoniac. Après refroidissement 700 ml d'eau sont ajoutés et le mélange est acidifié par de l'acide chlorhydrique 12N. Après extraction à l'éther, les phases éthérées sont regroupées, lavées à l'eau jusqu'à neutralité, séchées et évaporées. Le produit attendu est obtenu après distillation.
Rendement : 83 %
Point d'ébullition : 138-142°C (13 mm/Hg)
Indice de réfraction : n_{D}²⁷ = 1,458

### PREPARATION B : Acide 4,4-dicyclopropylbutanoïque

### STADE A : 1-Bromo-3,3-dicyclopropylpropane

A une solution refroidie à - 30°C de 22 g de 3,3-dicyclopropylpropanol (obtenu par réduction du composé décrit dans la préparation A en présence d'hydrure de lithium aluminium) dans 85 ml d'éther anhydre, 15,5 g de tribromure de phosphore sont ajoutés lentement et l'ensemble est coulé après retour à température ambiante dans 7 ml d'eau glacée. La phase organique est alors lavée par une solution saturée de bicarbonate de sodium puis à l'eau jusqu'à neutralité et enfin séchée et évaporée. Le produit attendu est obtenu après distillation sous vide.
Rendement : 60 %
Point d'ébullition : 101-105°C (18 mm/Hg)

### STADE B : 4,4-Dicyclopropylbutyronitrile

Le produit attendu est obtenu en faisant réagir le cyanure de potassium sur le composé décrit au stade précédent en milieu éthanol/eau.
Rendement : 73 %
Point d'ébullition : 114-117°C (17 mm/Hg)

### STADE C : Acide 4,4-dicyclopropylbutanoïque

Le produit attendu est obtenu en procédant comme au stade B de la préparation A avec le composé décrit au stade précédent.
Rendement : 74 %
Point d'ébullition : 170-172°C (16 mm/Hg)

### PREPARATION C : Acide 5,5-dicyclopropylpentanoïque

Le produit attendu est obtenu en utilisant le mode opératoire décrit dans Org. Synthese Coll. vol. II, 474 à partir du 1-bromo-3,3-dicyclopropylpropane décrit au stade A de la préparation B.
Rendement : 74 %
Point d'ébullition : 170-172°C (16 mm/Hg)

### PREPARATION D : Acide 6,6-dicyclopropylhexanoïque

Le produit attendu est obtenu en utilisant le procédé décrit dans la préparation B.

### PREPARATION E : Acide 5,5-dicyclopropylpropen-4-oïque

20 mmoles d'iodure de (carboxypropyl)triphénylphosphonium sont placées dans 100 ml de tétrahydrofurane anhydre. 40 ml d'une solution 1M de terbutylate de potassium dans le THF sont ajoutés goutte à goutte au mélange précédent sous atmosphère d'azote.

L'agitation est maintenue 30 minutes et une solution contenant 20 mmoles de dicyclopropylcétone dans 20 ml de THF anhydre est additionnée. L'ensemble est laissé 4 jours sous agitation à température ambiante. Après reprise par de l'eau, filtration de l'oxyde de triphénylphosphine, lavage par du dichlorométhane, la phase aqueuse est acidifiée par HCl6N et extraite au dichlorométhane. Les phases organiques sont lavées à l'eau, séchées et évaporées. Le produit attendu est obtenu après purification par chromatographie sur gel de silice en utilisant comme éluant un mélange dichlorométhane/éthanol (97/3).
Rendement : 50 %
Infrarouge (film liquide) v_{CO} = 1743 et 1711 cm⁻¹

### PREPARATION F : Acide 6,6-dicyclopropylhexen-5-oïque

Le produit attendu est obtenu en procédant comme dans la préparation E à partir de bromure de (carboxybutyl)triphénylphosphonium.
Rendement : 55 %
Infrarouge (film liquide) v_{CO}= 1709 cm⁻¹

### PREPARATION G : Acide 6,6-dicyclopropylhexen-4-oïque

Le produit attendu est obtenu en procédant comme dans la préparation E à partir de 2,2-dicyclopropylacétaldéhyde.
Rendement : 54 %
Infrarouge (nujol) v_{CO} = 1712 cm⁻¹

### PREPARATION H : Acide 3-trifluorométhylcinnamique

### STADE A : 3-Trifluorométhylcinnamate d'éthyle

450 mmoles d'hydrure de sodium sont placées dans 50 ml de diglyme anhydre. A ce mélange est ajouté, à température ambiante, une solution de 84 g de triéthylphosphonoacétate dans 300 ml de diglyme. L'agitation est maintenue jusqu'à la fin du dégazement d'hydrogène puis on ajoute une solution contenant 52 g de trifluoroacétophénone dans 100 ml de diglyme. Après 18 heures de reflux, l'ensemble est refroidi à 10°C et versé dans 500 ml d'eau. Après extraction à l'éther éthylique, séchage et évaporation, le produit attendu est purifié par distillation.
Rendement : 67 %
Point d'ébullition : 104-108°C (18 mm/Hg)

### STADE B : Acide 3-trifluorométhylcinnamique

Le produit attendu est obtenu par saponification du composé décrit au stade précédent.
Rendement : 92 %
Point de fusion : 95°C

### PREPARATION I : Acide 3-phényl-3-trifluorométhylpropanoïque

Le produit attendu est obtenu par hydrogénation catalytique en milieu éthanolique du composé décrit au stade A de la préparation A en utilisant le charbon palladié comme catalyseur puis saponification.
Rendement : 90 %
Point de fusion : 72°C

### PREPARATION J : Acide 4-phényl-4-trifluorométhylbutanoïque

Le produit attendu est obtenu en procédant comme dans la préparation B à partir de 3-phényl-3-trifluorométhylpropanol (lui-même obtenu par réduction du 3-phényl-3-trifluorométhylpropanoate d'éthyle).
Infrarouge (nujol) v_{CO} = 1728 cm⁻¹

### PREPARATION K : Acide 5-phényl-5-trifluorométhylpentanoïque

Le produit attendu est obtenu en procédant comme dans la préparation B à partir du 4-phényl-4-trifluorométhylbutanol (lui-même obtenu par réduction du composé obtenu dans la préparation J).
Point d'ébullition : 106-110°C (16 mm/Hg)

### PREPARATION L : Acide 5-cyclopentyl-5-trifluorométhylpentanoïque

Le produit attendu est obtenu en procédant comme dans la préparation B à partir du 4-cyclopentyl-4-trifluorométhylbutanol.

### PREPARATION M : Acide 3-(dicyclopropylméthylthio)propanoïque

### STADE A : 3-(dicyclopropylméthylthio)propionate de méthyle

Une solution contenant 6,8 g de dicyclopropylmercaptan (obtenu par réduction par le borohydrure de sodium de la dicyclopropylthiocétone elle-même préparée par action du réactif de LAWESSON sur la dicyclopropylcétone) dans 10 ml d'éthanol est ajoutée à une solution d'éthylate de sodium. Après 10 minutes d'agitation, on ajoute au mélange précédant 10 g de 3-bromopropionate de méthyle dans 10 ml d'éthanol. L'ensemble est maintenu 18 heures sous agitation à température ambiante. Le produit attendu est obtenu après filtration du bromure de sodium, évaporation et distillation.
Rendement : 66 %
Point d'ébullition : 158-160°C (16 mm/Hg)

### STADE B : Acide 3-(dicyclopropylméthylthio)propanoïque

Le produit attendu est obtenu par saponification du composé décrit au stade précédent.
Rendement : 84 %
Infrarouge (film liquide) v_{CO} = 1711 cm⁻¹

### PREPARATION N : Acide 3-dicyclopropylméthylaminocarbonylpropanoïque

Le produit attendu est obtenu par couplage peptidique de la dicyclopropylméthylamine avec le monosuccinate de méthyle selon la technique décrite par W. KONIG et R. GEIGER (Ber, 103, 788, 1970) puis saponification.
- Infrarouge (nujol) :: v_{CO} (acide) = 1703 cm⁻¹
v_{CO} (amide) = 1631 cm⁻¹

Les exemples 1-54 et 57-58 sont des exemples de préparation et/ou de comparaison

### EXEMPLE 1 : (3S)-2-Aza-2-tertbutyloxycarbonyl-3-[(pyrrolidin-1-yl) carbonyl]bicyclo[2.2.2]octane

En utilisant la technique de couplage peptidique (dicyclohexylcarbodiimide/hydroxybenzotriazole-DCC/HOBT) décrite par W. KONIG et R. GEIGER (Ber. 103, 788, 1970) et le diméthylformamide comme solvant, on prépare à partir de pyrrolidine et d'acide (3S)-2-aza-2-tertbutyloxycarbonyl bicyclo[2.2.2]octane-3-carboxylique, le produit attendu qui est purifié par cristallisation dans de l'acétate d'éthyle.
Rendement : 73 %
Point de fusion : 150-152°C
Pouvoir rotatoire : [α]_{D}²⁰ = - 10,5° (c = 1 %, EtOH)

| Microanalyse élémentaire : | | | |
|---|---|---|---|
| | C % | H % | N % |
| calculé | 66,20 | 9,15 | 9,08 |
| trouvé | 66,24 | 9,40 | 9,06 |

### EXEMPLE 2 : (3R)-2-Aza-2-tertbutyloxycarbonyl-3-[(pyrrolidin-1-yl) carbonyl]bicyclo[2.2.2]octane

Le produit attendu est obtenu selon le même procédé que celui décrit dans l'exemple 1 mais en utilisant l'isomère (3R) de l'acide 2-aza-2-tertbutyloxycarbonyl bicyclo[2.2.2]octane-3-carboxylique à la place de l'isomère (3S).
Point de fusion : 150-152°C

| Microanalyse élémentaire : | | | |
|---|---|---|---|
| | C % | H % | N % |
| calculé | 66,20 | 9,15 | 9,08 |
| trouvé | 66,93 | 9,15 | 9,18 |

Les exemples 3 à 6 ont été obtenus selon le procédé décrit dans l'exemple 1 en utilisant les produits de départ connus correspondants.

### EXEMPLE 3 : (1S, 3S, 4R)-2-Aza-2-tertbutyloxycarbonyl-3-[(pyrrolidin-1-yl)carbonyl]bicyclo[2.2.1]heptane

Point de fusion : 142-144°C

### EXEMPLE 4 : (3S)-2-Aza-2-tertbutyloxycarbonyl-3-[(3-azabicyclo[3.3.0] octan-3-yl)carbonyl]bicyclo[2.2.2]octane

Point de fusion : 154-156°C

| Microanalyse élémentaire : | | | |
|---|---|---|---|
| | C % | H % | N % |
| calculé | 68,93 | 9,26 | 8,04 |
| trouvé | 68,99 | 9,25 | 8,04 |

### EXEMPLE 5 : (2S, 3aS, 7aS)-1-(4-Phénylbutyryl)-2-[(pyrrolidin-1-yl) carbonyl]perhydroindole

| Microanalyse élémentaire : | | | |
|---|---|---|---|
| | C % | H % | N % |
| calculé | 67,05 | 9,38 | 8,69 |
| trouvé | 66,60 | 9,52 | 8,70 |

### EXEMPLE 6 : (1R, 3S, 4R)-2-Aza-2-tertbutyloxycarbonyl-3-[(pyrrolidin-1-yl)carbonyl]bicyclo[2,2,1]heptane

Point de fusion : 112°C

| Microanalyse élémentaire : | | | |
|---|---|---|---|
| | C % | H % | N % |
| calculé | 65,28 | 8,90 | 9,52 |
| trouvé | 65,28 | 8,82 | 9,68 |

### EXEMPLE 7 : (3S)-2-Aza-2-(4-phénylbutyryl)-3-[(pyrrolidin-1-yl)carbonyl] bicyclo[2.2.2]octane

### STADE A : (3S)-2-Aza-3-[(pyrrolidin-1-yl)carbonyl]bicyclo[2.2.2]octane

37 mmoles du composé décrit dans l'exemple 1 sont dissous dans 100 ml d'acétate d'éthyle anhydre. En maintenant la température à 20°C, on fait passer un courant d'acide chlorhydrique et l'agitation est maintenue 18 heures à température ambiante. Le solvant est évaporé et le résidu repris par 100 ml d'eau. L'insoluble est filtré et le filtrat, après alcalinisation par addition de bicarbonate de sodium, est amené à sec. Le résidu est enfin repris successivement par 100 ml d'éthanol, 100 ml de dichlorométhane puis 100 ml d'éther éthylique. Le produit attendu est obtenu après filtration des sels et évaporation.
Rendement : 89 %
Point de fusion : 117°C

### STADE B : (3S)-2-Aza-2-(4-phénylbutyryl)-3-[(pyrrolidin-1-yl)carbonyl] bicyclo[2.2.2]octane

Le produit attendu est obtenu selon le même procédé que celui décrit dans l'exemple 1 mais en utilisant l'acide 4-phénylbutyrique et le produit obtenu au stade A. Il est purifié par chromatographie sur silice en utilisant comme solvant d'élution un mélange dichlorométhane/méthanol (95/5) et cristallisé dans un mélange acétone-oxyde d'isopropyle.
Point de fusion : 77°C

| Microanalyse élémentaire : | | | |
|---|---|---|---|
| | C % | H % | N % |
| calculé | 74,54 | 8,53 | 7,90 |
| trouvé | 74,71 | 8,56 | 7,93 |

Les exemples 8 à 34 ont été obtenus selon le procédé décrit dans l'exemple 7 en utilisant les produits de départ correspondants connus ou décrits dans les préparations A à N.

### EXEMPLE 8 : (3R)-2-Aza-2-(4-phénylbutyryl)-3-[(pyrrolidin-1-yl)carbonyl] bicyclo[2.2.2]octane

Point de fusion : 70°C

### EXEMPLE 9 : (1S, 3S, 4R)-2-Aza-2-(4-phénylbutyryl)-3-[(pyrrolidin-1-yl) carbonyl]bicyclo[2.2.1]heptane

Infrarouge (nujol) : v_{CO} (amide) = 1650 cm⁻¹

### EXEMPLE 10 : (2S, 3aS, 7aS)-1-Tertbutyloxycarbonyl-2-[(pyrrolidin-1-yl) carbonyl]perhydroindole

| Microanalyse élémentaire : | | | |
|---|---|---|---|
| | C % | H % | N % |
| calculé | 74,96 | 8,75 | 7,60 |
| trouvé | 74,57 | 9,16 | 7,16 |

### EXEMPLE 11 : (3S)-2-(4-Phénylbutyryl)-3-[(pyrrolidin-1-yl)carbonyl]-1,2,3,4-tétrahydroisoquinoléine

| Microanalyse élémentaire : | | | |
|---|---|---|---|
| | C % | H % | N % |
| calculé | 76,56 | 7,50 | 7,44 |
| trouvé | 76,07 | 6,99 | 7,20 |

### EXEMPLE 12 : (3S)-2-Aza-2-[5,5-(dicyclopropyl)pentanoyl]-3-[(pyrrolidin-1-yl)carbonyl]bicyclo[2.2.2]octane

| Microanalyse élémentaire : | | | |
|---|---|---|---|
| | C % | H % | N % |
| calculé | 74,15 | 9,74 | 7,52 |
| trouvé | 74,22 | 10,15 | 7,62 |

### EXEMPLE 13 : (3S)-2-Aza-2-[6,6-(dicyclopropyl)hexanoyl]-3-[(pyrrolidin-1-yl)carbonyl]bicyclo[2.2.2]octane

Point de fusion : 86°C

### EXEMPLE 14 : (2S, 3aS, 7aS)-1-[5,5-(dicyclopropyl)pentanoyl]-2-[(pyrrolidin-1-yl)carbonyl]perhydroindole

| Microanalyse élémentaire : | | | |
|---|---|---|---|
| | C % | H % | N % |
| calculé | 74,57 | 9,91 | 7,25 |
| trouvé | 74,04 | 9,99 | 7,29 |

### EXEMPLE 15 : (2S, 3aS, 7aS)-1-[3,3-(dicyclopropyl)propionyl]-2-[(pyrrolidin-1-yl)carbonyl]perhydroindole

Point de fusion : 110-112°C

| Microanalyse élémentaire : | | | |
|---|---|---|---|
| | C % | H % | N % |
| calculé | 73,70 | 9,56 | 7,81 |
| trouvé | 73,36 | 9,61 | 8,14 |

### EXEMPLE 16 : (2S, 3aS, 7aS)-1-[4,4-(Dicyclopropyl)butyryl]-2-[(pyrrolidin-1-yl)carbonyl]perhydroindole

| Microanalyse élémentaire : | | | |
|---|---|---|---|
| | C % | H % | N % |
| calculé | 74,15 | 9,74 | 7,52 |
| trouvé | 73,81 | 9,64 | 7,68 |

### EXEMPLE 17 : (1S, 3S, 4R)-2-Aza-2-[5,5-(dicyclopropyl)pentanoyl]-3-[(pyrrolidin-1-yl)carbonyl]bicyclo[2,2,1]heptane

| Microanalyse élémentaire : | | | |
|---|---|---|---|
| | C % | H % | N % |
| calculé | 73,70 | 9,56 | 7,81 |
| trouvé | 73,93 | 9,55 | 7,82 |

### EXEMPLE 18 : (3S)-2-Aza-2-[6,6-(dicyclopropyl)hexèn-5-oyl]-3-[(pyrrolidin-1-yl)carbonyl]bicyclo[2.2.2]octane

- Infrarouge (nujol) :: v_{co} (amide) = 1676 et 1653 cm⁻¹ v_{c=c} = 1630 cm⁻¹

### EXEMPLE 19 : (3S)-2-Aza-2-[(E)-cinnamoyl]-3-[(pyrrolidin-1-yl)carbonyl] bicyclo[2.2.2]octane

Point de fusion : 184°C

### EXEMPLE 20 : (3S)-2-Aza-2-[(E)-4-phénylbutèn-3-oyl]-3-[(pyrrolidin-1-yl) carbonyl]bicyclo[2.2.2]octane

Point de fusion : 100-105°C

### EXEMPLE 21 : (3S)-2-Aza-2-[5,5-(dicyclopropyl)pentèn-4-oyl]-3-[(pyrrolidin-1-yl)carbonyl]bicyclo[2.2.2]octane

Infrarouge (nujol) : v_{co} (amide) = 1656 et 1637 cm⁻¹

### EXEMPLE 22 : (2S, 3aS, 7aS)-1-(3-Trifluorométhylcinnamoyl)-2-[(pyrrolidin-1-yl)carbonyl]perhydroindole

Point de fusion : 148°C

| Microanalyse élémentaire : | | | |
|---|---|---|---|
| | C % | H % | N % |
| calculé | 65,70 | 6,47 | 6,66 |
| trouvé | 65,66 | 6,64 | 6,75 |

### EXEMPLE 23 : (2S, 3aS, 7aS)-1-(3-Trifluorométhyl-3-phénylpropanoyl)-2-[(pyrrolidin-1-yl)carbonyl]perhydroindole, isomère α

Point de fusion : 134°C

| Microanalyse élémentaire : | | | |
|---|---|---|---|
| | C % | H % | N % |
| calculé | 65,38 | 6,91 | 6,63 |
| trouvé | 65,46 | 6,93 | 6,66 |

### EXEMPLE 24 : (2S, 3aS, 7aS)-1-(3-Trifluorométhyl-3-phénylpropanoyl)-2-[(pyrrolidin-1-yl)carbonyl]perhydroindole, isomère β

Point de fusion : 98°C

| Microanalyse élémentaire : | | | |
|---|---|---|---|
| | C % | H % | N % |
| calculé | 65,38 | 6,91 | 6,63 |
| trouvé | 65,42 | 6,99 | 6,51 |

### EXEMPLE 25 : (2S, 3aS, 7aS)-1-[(R,S)-4-Trifluorométhyl-4-phénylbutanoyl]-2-[(pyrrolidin-1-yl)carbonyl]perhydroindole

| Microanalyse élémentaire : | | | |
|---|---|---|---|
| | C % | H % | N % |
| calculé | 66,04 | 7,16 | 6,42 |
| trouvé | 65,78 | 7,18 | 6,25 |

### EXEMPLE 26 : (2S, 3aS, 7aS)-1-[(R,S)-5-Trifluorométhyl-5-phénylpentanoyl]-2-[(pyrrolidin-1-yl)carbonyl]perhydroindole

| Microanalyse élémentaire : | | | |
|---|---|---|---|
| | C % | H % | N % |
| calculé | 66,65 | 7,38 | 6,22 |
| trouvé | 66,97 | 7,60 | 6,14 |

### EXEMPLE 27 : (3S)-2-Aza-2-benzyloxycarbonyl-3-[(pyrrolidin-1-yl)carbonyl] bicyclo[2.2.2]octane

Point de fusion : 105°C

| Microanalyse élémentaire : | | | |
|---|---|---|---|
| | C % | H % | N % |
| calculé | 70,15 | 7,65 | 8,18 |
| trouvé | 70,04 | 7,50 | 8,17 |

### EXEMPLE 28 : (2S, 3aS, 7aS)-1-[3-(Dicyclopropylméthylthio)propanoyl]-2-[(pyrrolidin-1-yl)carbonyl]perhydroindole

| Microanalyse élémentaire : | | | | |
|---|---|---|---|---|
| | C % | H % | N % | S % |
| calculé | 68,28 | 8,97 | 6,92 | 7,92 |
| trouvé | 67,85 | 9,18 | 7,11 | 7,76 |

### EXEMPLE 29 : (3S)-2-Aza-2-[3-(N-Benzylanilinocarbonyl)propionyl]-3-[(pyrrolidin-1-yl)carbonyl]bicyclo[2.2.2]octane

| Microanalyse élémentaire : | | | |
|---|---|---|---|
| | C % | H % | N % |
| calculé | 73,54 | 7,45 | 8,87 |
| trouvé | 72,35 | 7,37 | 8,92 |

### EXEMPLE 30 : (3S)-2-Aza-2-[3-(dicyclopropylméthylaminocarbonyl)propionyl]-3-[(pyrrolidin-1-yl)carbonyl]bicyclo[2.2.2]octane

| Microanalyse élémentaire : | | | |
|---|---|---|---|
| | C % | H % | N % |
| calculé | 68,80 | 8,79 | 10,46 |
| trouvé | 68,96 | 8,81 | 10,47 |

### EXEMPLE 31 : (3S)-2-Aza-2-[((1S,2S)-2-phénylcycloprop-1-yl)carbonyl]-3-[(pyrrolidin-1-yl)carbonyl]bicyclo[2.2.2]octane

Pouvoir rotatoire : [a]_{D}^{21,5} = + 163,7° (c = 1 %, éthanol)

| Microanalyse élémentaire : | | | |
|---|---|---|---|
| | C % | H % | N % |
| calculé | 74,97 | 8,01 | 7,95 |
| trouvé | 75,05 | 8,10 | 7,71 |

### EXEMPLE 32 : (3S)-2-Aza-2-[((1R,2R)-2-phénylcycloprop-1-yl)carbonyl]-3-[(pyrrolidin-1-yl)carbonyl]bicyclo[2.2.2]octane

Pouvoir rotatoire : [a]_{D}^{21.5} = - 148,1° (c = 1 %, éthanol)

| Microanalyse élémentaire : | | | |
|---|---|---|---|
| | C % | H % | N % |
| calculé | 74,97 | 8,01 | 7,95 |
| trouvé | 74,73 | 8,10 | 7,67 |

### EXEMPLE 33 : (3S)-2-Aza-2-(6,6-dicyclopropylhexen-4-oyl)-3-[(pyrrolidin-1-yl)carbonyl]bicyclo[2.2.2]octane

Infrarouge (film liquide) v_{co} (amide) = 1639 cm⁻¹

### EXEMPLE 34 : (2S, 3aS, 7aS)-1-(5-cyclopentyl-5-trifluorométhylpentanoyl)-2-[(pyrrolidin-1-yl)carbonyl]perhydroindole

| Microanalyse élémentaire : | | | |
|---|---|---|---|
| | C % | H % | N % |
| calculé | 65,13 | 8,43 | 6,33 |
| trouvé | 65,05 | 8,43 | 6,34 |

Les exemples 35 à 43 ont été obtenus selon le procédé décrit dans l'exemple 1 mais en utilisant les produits de départ correspondants.

### EXEMPLE 35 : (2S, 3aS, 7aS)-1-Tertbutyloxycarbonyl-2-[(azetidin-1-yl) carbonyl]perhydroindole

Point de fusion : 130°C

| Microanalyse élémentaire : | | | |
|---|---|---|---|
| | C % | H % | N % |
| calculé | 66,20 | 9,15 | 9,08 |
| trouvé | 66,13 | 9,21 | 8,94 |

### EXEMPLE 36 : (2S, 3aS, 7aS)-1-Tertbutyloxycarbonyl-2-[(piperidin-1-yl) carbonyl]perhydroindole

Point de fusion : 132°C

| Microanalyse élémentaire : | | | |
|---|---|---|---|
| | C % | H % | N % |
| calculé | 67,82 | 9,59 | 8,33 |
| trouvé | 67,53 | 9,58 | 8,40 |

### EXEMPLE 37 : (2S, 3aS, 7aS)-1-Tertbutyloxycarbonyl-2-[(homopiperidin-1-yl)carbonyl]perhydroindole

Point de fusion : 122°C

| Microanalyse élémentaire : | | | |
|---|---|---|---|
| | C % | H % | N % |
| calculé | 68,54 | 9,78 | 7,99 |
| trouvé | 68,20 | 9,64 | 7,79 |

### EXEMPLE 38 : (2S, 3aS, 7aS)-1-Tertbutyloxycarbonyl-2-[(2-azabicyclo[2.2.2] octan-2-yl)carbonyl]perhydroindole

Point de fusion : 150-152°C

| Microanalyse élémentaire : | | | |
|---|---|---|---|
| | C % | H % | N % |
| calculé | 69,58 | 9,45 | 7,73 |
| trouvé | 69,58 | 9,56 | 8,07 |

### EXEMPLE 39 : (3S)-2-Aza-2-tertbutyloxycarbonyl-3-[(thiazolidin-3-yl) carbonyl]bicyclo[2.2.2]octane

Point de fusion : 174°C

| Microanalyse élémentaire : | | | | |
|---|---|---|---|---|
| | C % | H % | N % | S % |
| calculé | 58,87 | 8,03 | 8,58 | 9,82 |
| trouvé | 59,11 | 8,27 | 8,73 | 9,14 |

### EXEMPLE 40 : (3S)-2-Aza-2-tertbutyloxycarbonyl-3-[(pyrrolin-1-yl)carbonyl] bicyclo[2.2.2]octane

Point de fusion : 190°C

| Microanalyse élémentaire : | | | |
|---|---|---|---|
| | C % | H % | N % |
| calculé | 66,64 | 8,55 | 9,14 |
| trouvé | 66,76 | 8,52 | 9,24 |

### EXEMPLE 41 : (3S)-2-Aza-2-[5,5-(dicyclopropyl)pentanoyl]-3-[(thiazolidin-3-yl)carbonyl]bicyclo[2.2.2]octane

Point de fusion : 98-100°C
Infrarouge (nujol) v_{co} (amide) = 1635 cm⁻¹

### EXEMPLE 42 : (3S)-2-Aza-2-(4-phénylbutyryl)-3-[(imidazol-1-yl)carbonyl] bicyclo[2.2.2]octane

| Microanalyse élémentaire : | | | |
|---|---|---|---|
| | C % | H % | N % |
| calculé | 71,77 | 7,17 | 11,96 |
| trouvé | 71,95 | 7,22 | 12,03 |

### EXEMPLE 43 : (3S)-2-Aza-2-(4-phénylbutyryl)-2-[pyrazol-2-yl)carbonyl] bicyclo[2.2.2]octane

Infrarouge (nujol) v_{co} (amide) = 1637 cm⁻¹

### EXEMPLE 44 : (2S, 3aS, 7aS)-1-(5-phényl-5-trifluorométhylpentanoyl)-2-[(pyrrolidin-1-yl)carbonyl]perhydroindole, isomère α

### et EXEMPLE 45 : (2S, 3aS, 7aS)-1-(5-phényl-5-trifluorométhylpentanoyl)-2-[(pyrrolidin-1-yl)carbonyl]perhydroindole, isomère β

Les exemples 44 et 45 ont été préparés par séparation des isomères de l'exemple 26 par chromatographie sur gel de silice en utilisant comme éluant un mélange toluène-acétate d'éthyle 70/30. Les isomères ont été nommés a et p par ordre de sortie de colonne.

La pureté isomérique des deux composés a été contrôlée sur colonne chirale (Chiralpak ; longueur = 25 cm, diamètre intérieur = 4,6 mm, taille des particules = 10 µm) en utilisant comme solvant d'élution un mélange heptane/isopropanol/diéthylamine (85/15/0,05).
Exemple 44 : temps de rétention = 12,3 min
Exemple 45 : temps de rétention = 10,8 min

### EXEMPLE 46 : 3-[5,5-Dicyclopropylpentanoyl]-4-[2,5-dihydropyrrol-1-yl) carbonyl]thiazolidine

L'exemple 46 a été préparé selon le procédé décrit dans l'exemple 7.

Les exemples 47 à 58 ont été synthétisés selon le procédé décrit dans l'exemple 7 en utilisant les produits de départ correspondants.

### EXEMPLE 47 : (1S,3S,4R)-2-Aza-2-[((1S,2S)-2-phénylcycloprop-1-yl) carbonyl]-3-[(pyrrolidin-1-yl)carbonyl]bicyclo[2,2,1]heptane

Point de fusion : 175°C

| Microanalyse élémentaire : | | | |
|---|---|---|---|
| | C % | H % | N % |
| calculé | 74,53 | 7,74 | 8,28 |
| trouvé | 74,45 | 7,68 | 8,16 |

### EXEMPLE 48 : (1S,3S,4R)-2-Aza-2-[((1R,2R)-2-phénylcycloprop-1-yl) carbonyl]-3-[(pyrrolidin-1-yl)carbonyl]bicyclo[2.2,1]heptane

Point de fusion : 153°C

| Microanalyse élémentaire : | | | |
|---|---|---|---|
| | C % | H % | N % |
| calculé | 74,53 | 7,74 | 8,28 |
| trouvé | 74,44 | 7,92 | 7,95 |

### EXEMPLE 49 : (2S,3aS,7aS)-1-[((1S,2S)-2-phénylcycloprop-1-yl)carbonyl]-2-[(pyrrolidin-1-yl)carbonyl]perhydroindole

Point de fusion : 142°C

| Microanalyse élémentaire : | | | |
|---|---|---|---|
| | C % | H % | N % |
| calculé | 75,38 | 8,25 | 7,64 |
| trouvé | 75,37 | 8,27 | 7,65 |

### EXEMPLE 50 : (2S,3aS,7aS)-1-[((1R,2R)-2-phénylcycloprop-1-yl)carbonyl]-2-[(pyrrolidin-1-yl)carbonyl]perhydroindole

Point de fusion : 170°C

| Microanalyse élémentaire : | | | |
|---|---|---|---|
| | C % | H % | N % |
| calculé | 75,38 | 8,25 | 7,64 |
| trouvé | 75,04 | 8,32 | 7,58 |

### EXEMPLE 51 : (4R)-3-[((1S,2S)-2-phénylcycloprop-1-yl)carbonyl]-4-[(pyrrolidin-1-yl)carbonyl]thiazolidine

Point de fusion : 134°C

### EXEMPLE 52 : (4R)-3-[((1R,2R)-2-phénylcycloprop-1-yl)carbonyl]-4-[(pyrrolidin-1-yl)carbonyl]thiazolidine

Point de fusion : 200°C

Les composés des exemples 31, 47, 49, 51, 53, 55, 57 et 32, 48, 50, 52, 54, 56, 58 peuvent également être obtenus à partir des acides 2-phénylcycloprop-1-yl carboxyliques de configuration respectives (1S,2S) et (1R,2R) préparés selon le procédé décrit dans "Optical resolution for Chemical compounds" (vol.2, part I / Optical Resolution information center, Manhattan College, Riverdale - NY 10471, USA).

### EXEMPLE 53 : (2S,3aS,7aS)-1-[((1S,2S)-2-phénylcycloprop-1-ylcarbonyl]-2-[(2,5-dihydropyrrolin-1-yl)carbonyl]perhydroindole

### EXEMPLE 54 : (2S,3aS,7aS)-1-[((1R,2R)-2-phénylcycloprop-1-ylcarbonyl]-2-[(2,5-dihydropyrrolin-1-yl)carbonyl]perhydroindole

Infrarouge (nujol) v_{CO} (amide) et v_{C=C} = entre 1660 et 1624 cm⁻¹

### EXEMPLE 55 : (2S,3aS,7aS)-1-[((1S,2S)-2-phénylcycloprop-1-yl)carbonyl]-2-[(1,3-thiazolidin-3-yl)carbonyl]perhydroindole

| Microanalyse élémentaire : | | | | |
|---|---|---|---|---|
| | C % | H % | N % | S % |
| calculé | 68,72 | 7,34 | 7,28 | 8,34 |
| trouvé | 68,64 | 7,47 | 7,98 | 8,10 |

### EXEMPLE 56 : (2S,3aS,7aS)-1-[((1R,2R)-2-phénylcycloprop-1-yl)carbonyl]-2-[(1,3-thiazolidin-3-yl)carbonyl]perhydroindole

### EXEMPLE 57 : (2S,3aS,7aS)-[(1S,2S)-2-phénylcycloprop-1-yl)carbonyl]-2-[(pyrrol-1-yl)carbonyl]perhydroindole

### EXEMPLE 58 : (2S,3aS,7aS)-[(1R,2R)-2-phénylcycloprop-1-yl)carbonyl]-2-[(pyrrol-1-yl)carbonyl]perhydroindole

| Microanalyse élémentaire : | | | |
|---|---|---|---|
| | C % | H % | N % |
| calculé | 76,21 | 7,23 | 7,73 |
| trouvé | 75,68 | 7,11 | 7,41 |

### ETUDE PHARMACOLOGIQUE DES DERIVES DE L'INVENTION

### EXEMPLE 59 : Mesure de l'activité anti-prolyl-endopeptidase

La méthode de YOSHIMOTO et TSURU (Agr. Biol. Chem., 42, 2417, 1978) a été utilisée. La prolyl-endopeptidase est isolée de Flavobacterium meningosepticum. Le substrat enzymatique est la Z-Gly-Pro-pNitroaniline.

Un mélange de tampon phosphate 0,1 M (pH 7 ; 0,99 ml), de substrat 2 M (0,25 ml) et d'une solution du composé inhibiteur à tester (0,01 ml) est incubé à 37°C durant 5 minutes. Puis 0,1 ml d'une solution de prolyl-endopeptidase (0,5 U/ml) est ajouté et le mélange est laissé 10 minutes à 37°C. La réaction enzymatique est stoppée par addition de 2 ml d'une solution de Triton X-100-Tampon acétate 1 M (pH 4). Après repos à température ambiante, l'absorbance est mesurée à 410 nm.

Une mesure témoin est réalisée en remplaçant la solution du composé à tester par le même volume de tampon phosphate. Le pourcentage d'inhibition de l'enzyme est calculé à partir de cette mesure témoin. Pour chaque composé testé, la concentration inhibant de 50 % la prolyl-endopeptidase (IC50) est déterminée.

Les composés de l'invention ont été comparés aux deux composés de référence connus pour leur forte activité inhibitrice de la prolyl-endopeptidase et décrits initialement dans ce but : ONO1603 (EP 345 428) et N-(4-phénylbutanoyl)-L-thiaprolylpyrrolidinéimide (exemple 3 : EP 321 956) appelé ci-dessous ZERIA.

Les dérivés de l'invention exercent une forte activité inhibitrice de la prolyl-endopeptidase, supérieure aux composés de référence comme l'indique le tableau des IC50 :

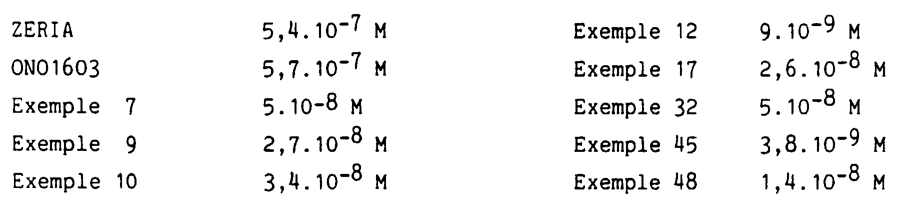

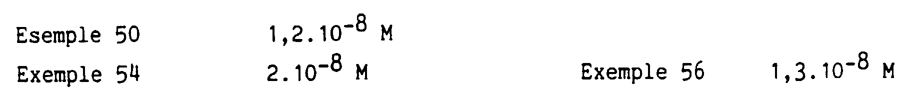

### EXEMPLE 60 :

La mesure de l'inhibition de la prolyl-endopeptidase a été réalisée in vitro et in vivo dans le cortex cérébral du Rat, grâce à un substrat proche d'un peptide naturellement clivé par la prolyl-endopeptidase, la Thyrotropin-Releasing Hormone (TRH).

Un extrait de cortex cérébral est préparé en broyant le tissu dans un tampon phosphate (NaH₂PO4 25 mM ; DTT 2 mM ; EDTA-2K 0,5 mM) à raison de 5 ml/g.

Après centrifugation, 200 µl de surnageant sont incubés 15 minutes à 37°C dans un volume final de 2,1 ml auquel sont ajoutés 250 µl de substrat (TRH-pNitroaniline) pour une nouvelle incubation de 20 minutes suivie d'une lecture immédiate d'absorbance à 410 nm.

Le produit testé est soit ajouté à l'extrait de cortex avant la première incubation (détermination de l'IC50 in vitro) soit administré par voie IP 30 minutes avant la préparation de l'extrait tissulaire.

Dans les conditions du test, les composés de l'invention inhibent fortement la prolyl-endopeptidase du cortex cérébral de Rat tant in vitro qu'après administration in vivo. Cette activité est là encore très supérieure à celle des composés de référence.

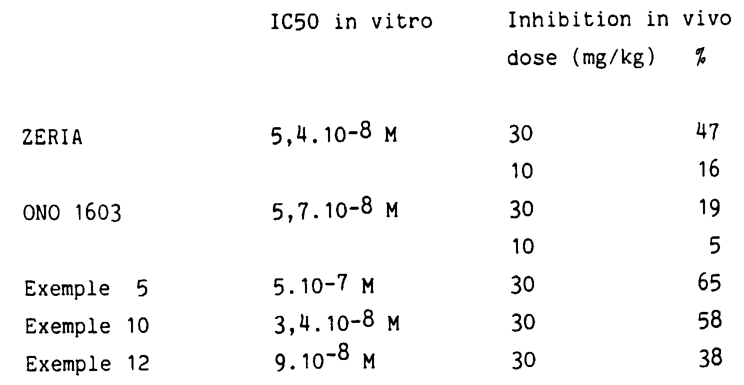

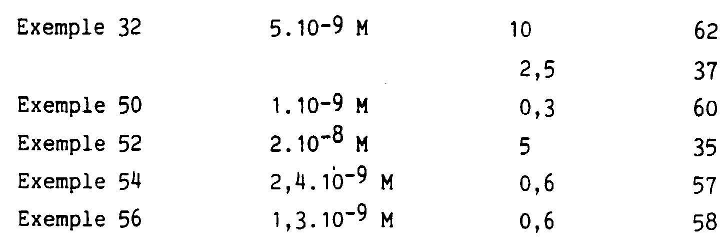

### EXEMPLE 61 :

La durée d'action des composés vis-à-vis de l'inhibition in vivo de la prolyl-endopeptidase dans le cortex cérébral du Rat a été estimée selon la même méthodologie que dans l'exemple 58, en administrant par voie IP les composés étudiés à divers temps avant la préparation de l'extrait tissulaire. Le temps pour lequel l'effet inhibiteur est égal à 50 % de l'effet maximum (mesuré à 30 minutes) a été calculé (t 1/2).

De même, la biodisponibilité orale des composés de l'invention a été estimée en les administrant par voie IP ou orale 30 minutes ou 60 minutes avant la mesure de l'effet inhibiteur. L'index de biodisponibilité orale (IBO) a été déterminé en divisant l'effet inhibiteur (%) mesuré après administration orale par celui mesuré après administration IP. Les composés les plus actifs de l'invention exercent leurs effets selon une durée d'action cérébrale bien plus longue que celle des composés de référence et avec une biodisponibilité orale bien plus importante, ce qui leur confère un intérêt thérapeutique prépondérant.

| | dose (mg/kg) | t 1/2 | IBO |
|---|---|---|---|
| ZERIA | 30 | 1 heure | 0,30 |
| ONO 1603 | 30 | 1 heure | <0,30 |
| Exemple 5 | 15 | 2 heures | 0,40 |
| Exemple 10 | 30 | 2 heures | 0,40 |
| Exemple 32 | 2,5 | 7 heures | 0,75 |
| Exemple 50 | 0,15 | 7 heures | 0,85 |
| Exemple 54 | 0,6 | 7 heures | 0,80 |
| Exemple 56 | 0,6 | 7 heures | 0,80 |

### COMPOSITION PHARMACEUTIQUE

### EXEMPLE 62 : Comprimé : Formule de préparation pour 1000 comprimés dosés à 10 mg

| | |
|---|---|
| Composé de l'exemple 1 | 10 g |
| Hydroxypropyl cellulose | 2 g |
| Amidon de blé | 10 g |
| Lactose | 100 g |
| Stéarate de magnésium | 3 g |
| Talc | 3 g |

## Revendications (Revendications pour l'(les) Etat(s) contractant(s) suivant(s): AT, BE, CH, DE, DK, FR, GB, IT, LI, LU, NL, PT, SE, IE)

1. Composé de formule (I): 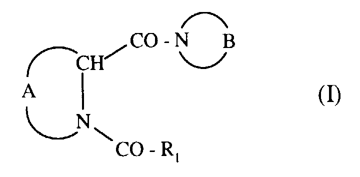 dans laquelle :
A représente avec les atomes de carbone et d'azote auquel il est attaché l'hétérocyclique suivant : 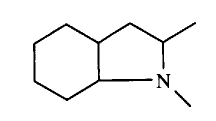
B représente avec l'atome d'azote auquel il est attaché l'hétérocycle suivant : 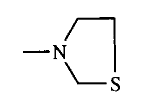
R₁ représente un groupement phénylcyclopropyl,
ses énantiomères, diastéréoisomères et épimères ainsi que ses sels d'addition à un acide pharmaceutiquement acceptable.

2. Composé de formule (I) selon la revendication 1 qui est le (2S,3aS,7aS)-1-[((1R,2R)-2-phénylcycloprop-1-yl)carbonyl]-2-[(1,3-thiazolidin-3-yl)carbonyl]perhydroindole.

3. Procédé de préparation des composés de formule (I) caractérisé en ce que l'on fait réagir un acide de formule (II), dont on a éventuellement séparé les isomères par une technique classique de séparation : 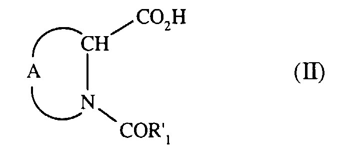 dans laquelle A a la même signification que dans la formule (I) et R'₁ représente un groupement alkoxy (C₁-C₆) linéaire ou ramifié ou benzyloxy,
sur une amine de formule (III), dont on a éventuellement séparé les isomères par une technique classique de séparation, selon une technique de couplage peptidique : 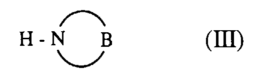 dans laquelle B a la même signification que dans la formule (I),
pour conduire au composé de formule (I/a), cas particulier des composés de formule (I) : 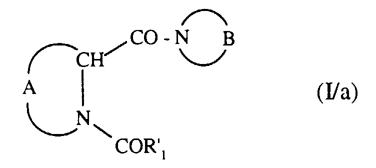 dans laquelle A, B et R'₁ ont la même signification que précédemment,
que l'on déprotège, si on le souhaite, par une technique classique de déprotection, pour conduire à l'amine de formule (IV) : 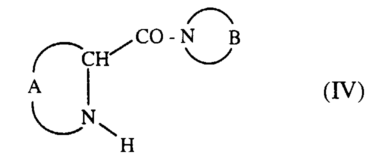 dans laquelle A et B ont la même signification que dans la formule (I),
que l'on fait réagir avec un acide de formule (V) en présence d'un agent de couplage classique de la synthèse peptidique :
HO₂C-R"₁ (V)
dans laquelle :
R"₁ a la même signification que R₁ à l'exception du cas où R₁ représente R'₁
pour conduire au composé de formule (I/b), cas particulier des composés de formule (I) : 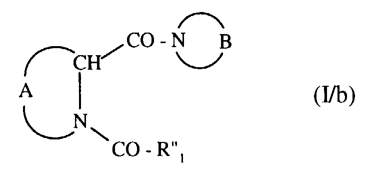 dans laquelle A, B et R"₁ ont la même signification que précédemment,
dérivés de formule (I/a) et (I/b) que l'on purifie, le cas échéant, par une technique classique de purification, dont on sépare, si on le souhaite, les isomères par une technique classique de séparation et que l'on transforme, si nécessaire, en leurs sels d'addition à un acide pharmaceutiquement acceptable.

4. Compositions pharmaceutiques contenant comme principe actif au moins un composé selon l'une quelconque des revendications 1 ou 2, seul ou en combinaison avec un ou plusieurs excipients ou véhicules inertes, non toxiques, pharmaceutiquement acceptables.

5. Compositions pharmaceutiques selon la revendication 4 contenant au moins un principe actif selon l'une des revendications 1 ou 2 utiles en tant qu'inhibiteurs de prolyl-endopeptidase pour le traitement des désordres mnésiques et cognitifs et des troubles neurocomportementaux associés à la dépression, à l'anxiété, au vieillissement et aux maladies dégénératives du système nerveux, aigües ou chroniques comme la maladie d'Alzheimer, de Pick, de Korsakoff, l'accident vasculaire cérébral, le traumatisme spinal ou la sclérose amyotrophique latérale.

## Revendications (Revendications pour l'(les) Etat(s) contractant(s) suivant(s): ES, GR)

1. Procédé de préparation du composé de formule (I) : 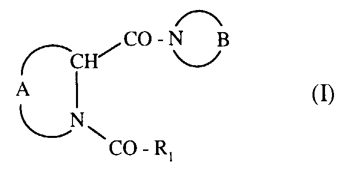 dans laquelle :
A représente avec les atomes de carbone et d'azote auquel il est attaché l'hétérocyclique suivant: 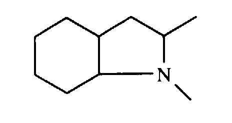
B représente avec l'atome d'azote auquel il est attaché l'hétérocycle suivant : 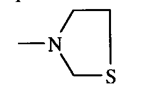
R₁ représente un groupement phénylcyclopropyl,
ses énantiomères, diastéréoisomères et épimères ainsi que ses sels d'addition à un acide pharmaceutiquement acceptable,
caractérisé en ce que l'on fait réagir un acide de formule (II), dont on a éventuellement séparé les isomères par une technique classique de séparation : 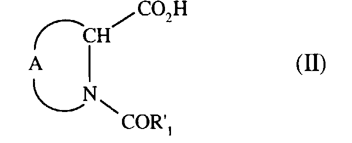 dans laquelle A a la même signification que dans la formule (I) et R'₁ représente un groupement alkoxy (C₁-C₆) linéaire ou ramifié ou benzyloxy, sur une amine de formule (III), dont on a éventuellement séparé les isomères par une technique classique de séparation, selon une technique de couplage peptidique : 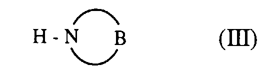 dans laquelle B a la même signification que dans la formule (I),
pour conduire au composé de formule (I/a), cas particulier des composés de formule (I) : 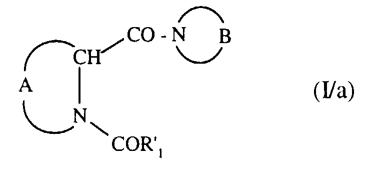 dans laquelle A, B et R'₁ ont la même signification que précédemment,
que l'on déprotège, si on le souhaite, par une technique classique de déprotection, pour conduire à l'amine de formule (IV) : 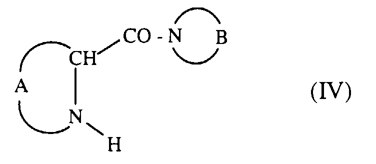 dans laquelle A et B ont la même signification que dans la formule (I),
que l'on fait réagir avec un acide de formule (V) en présence d'un agent de couplage classique de la synthèse peptidique :
HO₂C - R"₁ (V)
dans laquelle :
R"₁ a la même signification que R₁ à l'exception du cas où R₁ représente R'₁
pour conduire au composé de formule (I/b), cas particulier des composés de formule (I) : 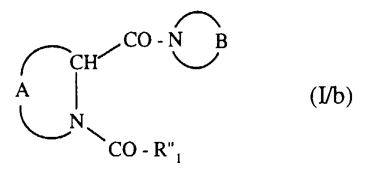 dans laquelle A, B et R"₁ ont la même signification que précédemment,
dérivés de formule (I/a) et (I/b) que l'on purifie, le cas échéant, par une technique classique de purification, dont on sépare, si on le souhaite, les isomères par une technique classique de séparation et que l'on transforme, si nécessaire, en leurs sels d'addition à un acide pharmaceutiquement acceptable.

2. Procédé de préparation du composé de formule (I) selon la revendication 1 qui est le (2S,3aS,7aS)-1-[((1R,2R)-2-phénylcycloprop-1-yl)carbonyl]-2-[(1,3-thiazolidin-3-yl) carbonyl]perhydroindole.

## Patentansprüche (Patentansprüche für folgende(n) Vertragsstaat(en): AT, BE, CH, DE, DK, FR, GB, IT, LI, LU, NL, PT, SE, IE)

1. Verbindung der Formel (I): 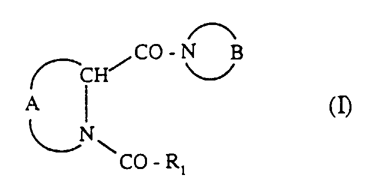 in der:
A zusammen mit dem Kohlenstoff- und Stickstoffatom, an die es gebunden ist, den folgenden Heterocyclus darstellt: 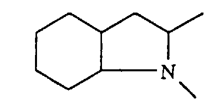
B zusammen mit dem Stickstoffatom, an das es gebunden ist, den folgenden Heterocyclus darstellt: 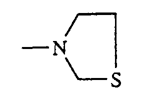 und
R₁ eine Phenylcyclopropylgruppe bedeutet,
deren Enantiomere, Diastereoisomere und Epimere sowie deren Additionssalze mit einer pharmazeutisch annehmbaren Säure.

2. Verbindung der Formel (I) nach Anspruch 1, nämlich (2S,3aS,7aS)-1-[((1R,2R)-2-Phenylcycloprop-1-yl)-carbonyl]-2-[(1,3-thiazolidin-3-yl)-carbonyl]-perhydroindol.

3. Verfahren zur Herstellung der Verbindungen der Formel (I), **dadurch gekennzeichnet,** daß man eine Säure der Formel (II), die man gegebenenfalls mit Hilfe einer klassischen Trennmethode in ihre Isomeren aufgetrennt hat: 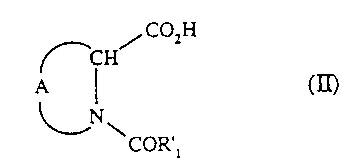 in der A die bezüglich der Formel (I) angegebenen Bedeutungen besitzt und R'₁ eine geradkettige oder verzweigte (C₁-C₆)-Alkoxygruppe oder eine Benzyloxygruppe darstellt,
mit einem Amin der Formel (III), das man gegebenenfalls mit Hilfe einer klassischen Trennmethode in die Isomeren aufgetrennt hat, mit Hilfe einer Peptidkupplungstechnik umsetzt: 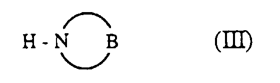 in der B die bezüglich der Formel (I) angegebenen Bedeutungen besitzt,
zur Bildung der Verbindung der Formel (I/a), einem Sonderfall der Verbindungen der Formel (I): 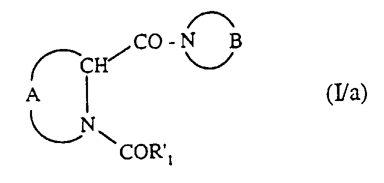 in der A. B und R'₁ die oben angegebenen Bedeutungen besitzen,
von welcher man gewünschtenfalls die Schutzgruppe mit Hilfe einer klassischen Schutzgruppenabspaltungstechnik abspaltet zur Bildung des Amins der Formel (IV): 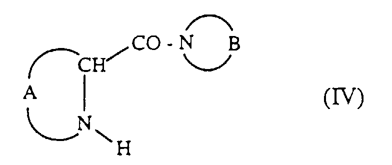 in der A und B die bezüglich der Formel (I) angegebenen Bedeutungen besitzen. welches man in Gegenwart eines klassischen Kupplungsmittels der Peptidsynthese mit einer Säure der Formel (V) umsetzt:
HO₂C - R"₁ (V)
in der:
R"₁ die gleichen Bedeutungen besitzt wie R₁, mit Ausnahme des Falls, da R₁ R'₁ darstellt,
zur Bildung der Verbindung der Formel (I/b), einem Sonderfall der Verbindungen der Formel (I): 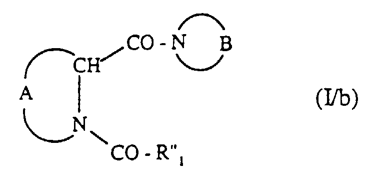 in der A, B und R"₁ die oben angegebenen Bedeutungen besitzen,
welche Derivate der Formel (I/a) und (I/b) man gegebenenfalls mit Hilfe einer klassischen Reinigungsmethode reinigt, gewünschtenfalls mit Hilfe einer klassischen Trennmethode in die Isomeren auftrennt und erforderlichenfalls in ihre Additionssalze mit einer pharmazeutisch annehmbaren Säure umwandelt.

4. Pharmazeutische Zubereitungen enthaltend als Wirkstoff mindestens eine Verbindung nach irgendeinem der Ansprüche 1 oder 2 allein oder in Kombination mit einem oder mehreren inerten, nichttoxischen, pharmazeutisch annehmbaren Trägermaterialien oder Bindemitteln.

5. Pharmazeutische Zubereitungen nach Anspruch 4 enthaltend mindestens einen Wirkstoff nach einem der Ansprüche 1 oder 2 als Inhibitoren der Prolyl-Endopeptidase zur Behandlung von Gedächtnis- und Erkenntnisstörungen und von Störungen des Neuroverhaltens als Folge der Depression, der Angst, des Alterns und von degenerativen akuten oder chronischen Erkrankungen des Nervensystems, wie der Alzheimerschen Krankheit, der Pickschen Krankheit, der Korsakoffschen Krankheit, von Gehirngefäßvorfällen, Rückenmarkstraumata oder lateraler amyotropher Sklerose.

## Patentansprüche (Patentansprüche für folgende(n) Vertragsstaat(en): ES, GR)

1. Verfahren zur Herstellung der Verbindung der Formel (I): 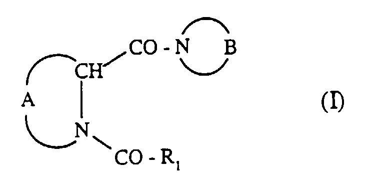 in der:
A zusammen mit dem Kohlenstoff- und Stickstoffatom, an die es gebunden ist, den folgenden Heterocyclus darstellt: 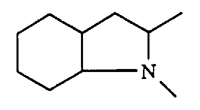
B zusammen mit dem Stickstoffatom, an das es gebunden ist, den folgenden Heterocyclus darstellt: 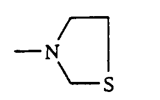 und
R₁ eine Phenylcyclopropylgruppe bedeutet,
von deren Enantiomeren, Diastereoisomeren und Epimeren sowie von deren Additionssalzen mit einer pharmazeutisch annehmbaren Säure,
**dadurch gekennzeichnet**, daß man eine Säure der Formel (II), die man gegebenenfalls mit Hilfe einer klassischen Trennmethode in ihre Isomeren aufgetrennt hat: 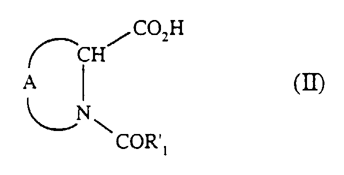 in der A die bezüglich der Formel (I) angegebenen Bedeutungen besitzt und R'₁ eine geradkettige oder verzweigte (C₁-C₆)-Alkoxygruppe oder eine Benzyloxygruppe darstellt,
mit einem Amin der Formel (III), das man gegebenenfalls mit Hilfe einer klassischen Trennmethode in die Isomeren aufgetrennt hat, mit Hilfe einer Peptidkupplungstechnik umsetzt: 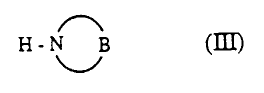 in der B die bezüglich der Formel (I) angegebenen Bedeutungen besitzt,
zur Bildung der Verbindung der Formel (I/a), einem Sonderfall der Verbindungen der Formel (I): 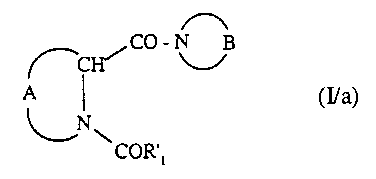 in der A. B und R'₁ die oben angegebenen Bedeutungen besitzen,
von welcher man gewünschtenfalls die Schutzgruppe mit Hilfe einer klassischen Schutzgruppenabspaltungstechnik abspaltet zur Bildung des Amins der Formel (IV): 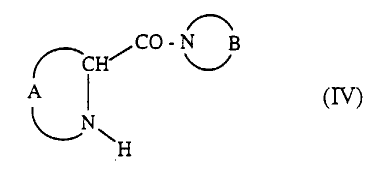 in der A und B die bezüglich der Formel (I) angegebenen Bedeutungen besitzen, welches man in Gegenwart eines klassischen Kupplungsmittels der Peptidsynthese mit einer Säure der Formel (V) umsetzt:
HO₂C - R"₁ (V)
in der:
R"₁ die gleichen Bedeutungen besitzt wie R₁, mit Ausnahme des Falls, da R₁ R'₁ darstellt,
zur Bildung der Verbindung der Formel (I/b), einem Sonderfall der Verbindungen der Formel (I): 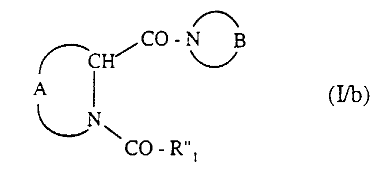 in der A, B und R"₁ die oben angegebenen Bedeutungen besitzen,
welche Derivate der Formel (I/a) und (I/b) man gegebenenfalls mit Hilfe einer klassischen Reinigungsmethode reinigt, gewünschtenfalls mit Hilfe einer klassischen Trennmethode in die Isomeren auftrennt und erforderlichenfalls in ihre Additionssalze mit einer pharmazeutisch annehmbaren Säure umwandelt.

2. Verfahren zur Herstellung der Verbindung der Formel (I) nach Anspruch 1, nämlich von (2S,3aS,7aS)-1-[((1R,2R)-2-Phenylcycloprop-1-yl)-carbonyl]-2-[[1,3-thiazolidin-3-yl)-carbonyl]perhydroindol.

## Claims (Claims for the following Contracting State(s): AT, BE, CH, DE, DK, FR, GB, IT, LI, LU, NL, PT, SE, IE)

1. Compound of formula (I) : 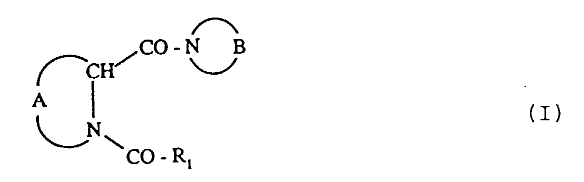 wherein :
A represents, together with the carbon and nitrogen atoms to which it is attached, the following heterocycle : 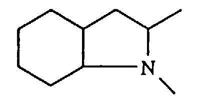
B represents, together with the nitrogen atom to which it is attached, the following heterocycle : 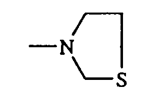
R₁ represents a phenylcyclopropyl group,
its enantiomers, diastereoisomers and epimers, and also the addition salts thereof with a pharmaceutically acceptable acid.

2. Compound of formula (I) according to claim 1 which is (2S,3aS,7aS)-1-[((1R,2R)-2-phenylcycloprop-1-yl)-carbonyl]-2-[(1,3-thiazolidin-3-yl)carbonyl]perhydroindole.

3. Process for the preparation of compounds of formula (I), characterised in that an acid of formula (II) 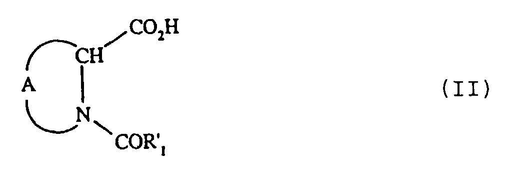 wherein A is as defined for formula (I) and R'₁ represents a straight-chain or branched (C₁-C₆)alkoxy group or a benzyloxy group, which has optionally been separated into its isomers by a conventional separation technique, is reacted according to a peptide coupling technique with an amine of formula (III) : 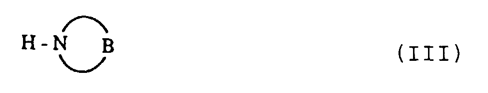 wherein B is as defined for formula (I), which has optionally been separated into its isomers by a conventional separation technique,
to yield the compound of formula (I/a) 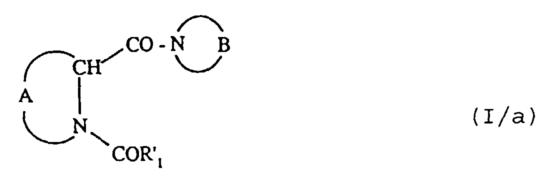 wherein A, B and R'₁ are as defined above, a particular case of compounds of formula (I),
which compound of formula (I/a) is deprotected, if desired, by a conventional deprotection technique to yield the amine of formula (IV) : 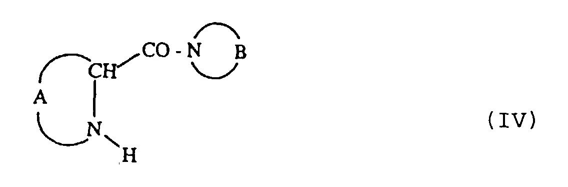 wherein A and B are as defined for formula (I),
which is reacted with an acid of formula (V) :
HO₂C-R"₁ (V)
wherein :
R"₁ has the same meaning as R₁ with the exception of the case in which R₁ represents R'₁,
in the presence of a conventional peptide synthesis coupling agent
to yield the compound of formula (I/b) 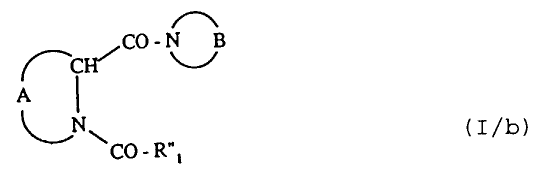 wherein A, B and R"₁ are as defined above, a particular case of compounds of formula (I),
which compounds of formula (I/a) and (I/b) are purified, where necessary, by a conventional purification technique, are separated, if desired, into their isomers by a conventional separation technique and are converted, if necessary, into their addition salts with a pharmaceutically acceptable acid.

4. Pharmaceutical compositions comprising as active ingredient at least one compound according to either claim 1 or claim 2, alone or in combination with one or more pharmaceutically acceptable, inert, non-toxic excipients or carriers.

5. Pharmaceutical compositions according to claim 4 comprising at least one active ingredient according to either claim 1 or claim 2 for use as prolyl-endopeptidase inhibitors in the treatment of mnesic and cognitive disorders and neurobehavioural disorders associated with depression, anxiety, ageing and with acute or chronic degenerative diseases of the central nervous system, such as Alzheimer's disease, Pick's disease, Korsakoff's disease, cerebral vascular accident, spinal trauma or lateral amyotrophic sclerosis.

## Claims (Claims for the following Contracting State(s): ES, GR)

1. Process for the preparation of a compound of formula (I) : 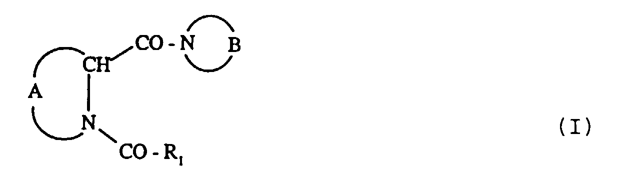 wherein :
A represents, together with the carbon and nitrogen atoms to which it is attached, the following heterocycle : 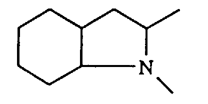
B represents, together with the nitrogen atom to which it is attached, the following heterocycle : 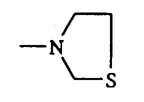
R₁ represents a phenylcyclopropyl group,
its enantiomers, diastereoisomers and epimers, and also the addition salts thereof with a pharmaceutically acceptable acid,
characterised in that an acid of formula (II) 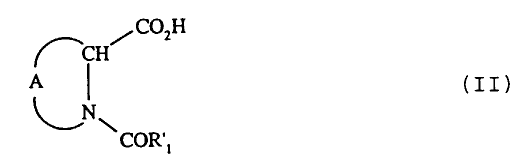 wherein A is as defined for formula (I) and R'₁ represents a straight-chain or branched (C₁-C₆)alkoxy group or a benzyloxy group, which has optionally been separated into its isomers by a conventional separation technique, is reacted according to a peptide coupling technique with an amine of formula (III) : 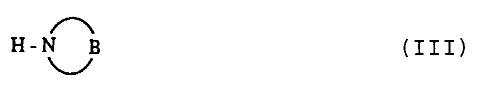 wherein B is as defined for formula (I), which has optionally been separated into its isomers by a conventional separation technique,
to yield the compound of formula (I/a) 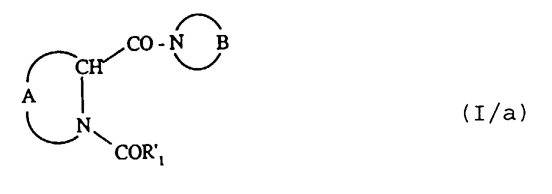 wherein A, B and R'₁ are as defined above, a particular case of compounds of formula (I),
which compound of formula (I/a) is deprotected, if desired, by a conventional deprotection technique to yield the amine of formula (IV) : 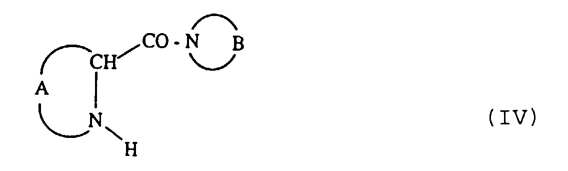 wherein A and B are as defined for formula (I),
which is reacted with an acid of formula (V) :
HO₂C-R"₁ (V)
wherein :
R"₁ has the same meaning as R₁ with the exception of the case in which R₁ represents R'₁,
in the presence of a conventional peptide synthesis coupling agent
to yield the compound of formula (I/b) 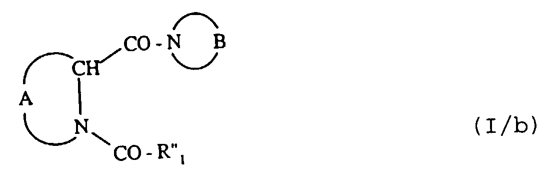 wherein A, B and R"₁ are as defined above, a particular case of compounds of formula (I),
which compounds of formula (I/a) and (I/b) are purified, where necessary, by a conventional purification technique, are separated, if desired, into their isomers by a conventional separation technique and are converted, if necessary, into their addition salts with a pharmaceutically acceptable acid.

2. Process for the preparation of the compound of formula (I) according to claim 1 that is (2S,3aS,7aS)-1-[((1R,2R)-2-phenylcycloprop-1-yl)carbonyl]-2-[(1,3-thiazolidin-3-yl)carbonyl]perhydroindole.
